Europäisches Patentamt

European Patent Office

Office européen des brevets

(19)

(11) Numéro de publication: **0 415 837 A2**

# DEMANDE DE BREVET EUROPEEN

(21) Numéro de dépôt: 90402369.4

(22) Date de dépôt: 27.08.90

(51) Int. Cl.5: **A61B 17/56**

(30) Priorité: 29.08.89 FR 8911333

(43) Date de publication de la demande:
06.03.91 Bulletin 91/10

(84) Etats contractants désignés:
BE CH DE ES GB IT LI NL

(71) Demandeur: **COMMISSARIAT A L'ENERGIE ATOMIQUE Etablissement de Caractère Scientifique Technique et Industriel**
31/33, rue de la Fédération
F-75015 Paris(FR)

(72) Inventeur: **Doucet, Michel**
**20 rue Georges Bizet**
**F-37260 Monts(FR)**
Inventeur: **Rideau, Joel**
**7 rue de la Croix de Monts**
**F-37260 Monts(FR)**

(74) Mandataire: **Mongrédien, André et al**
**c/o BREVATOME 25, rue de Ponthieu**
**F-75008 Paris(FR)**

(54) **Dispositif de positionnement et de guidage de la lame d'une scie chirurgicale.**

(57) L'invention permet de positionner une lame de scie chirurgicale par rapport à un os à scier, sans mettre en contact cette lame de scie avec un guide quelconque.

L'invention consiste à équiper le bloc de sciage (8) d'une coulisse rétractable (20), et à équiper le guide de positionnement (16) d'une fente (12) orientée de façon déterminée, de sorte que, lorsque la coulisse (20) est introduite dans la fente (12), la lame de scie est positionnée pour effectuer le sciage de l'os (2). Le guide (16) est orientable en translation et en rotation. La coulisse (20) est élastiquement rétractable pour permettre à l'opérateur, lorsqu'il appuie sur le bloc (8), de faire pénétrer la lame de scie (10) dans l'os (2).

Application particulière à la résection du plateau tibial.

FIG. 3

EP 0 415 837 A2

## DISPOSITIF DE POSITIONNEMENT ET DE GUIDAGE DE LA LAME D'UNE SCIE CHIRURGICALE

L'invention ressort du domaine de la chirurgie prothétique, et en particulier de la pose d'une prothèse du genou. L'invention concerne un dispositif de positionnement et de guidage de la lame d'une scie chirurgicale. La réalisation décrite est adaptée pour la résection du plateau tibial.

Pour l'implantation d'une prothèse totale ou partielle du genou, le chirurgien est amené à effectuer de nombreuses coupes osseuses. Ces coupes doivent répondre à des critères précis, pour que l'emboîtement de la prothèse soit sans défaut. Ces critères concernent la géométrie, l'encombrement, la rapidité, la sécurité et la température qui doit être inférieure à 55° C.

L'outillage connu et utilisé jusqu'à présent est composé de deux parties distinctes. La première est un outil coupant, en l'occurrence une lame de scie, mue par un ensemble-moteur pneumatique ou électrique. La seconde est un assortiment de guides et de supports pour la lame de scie.

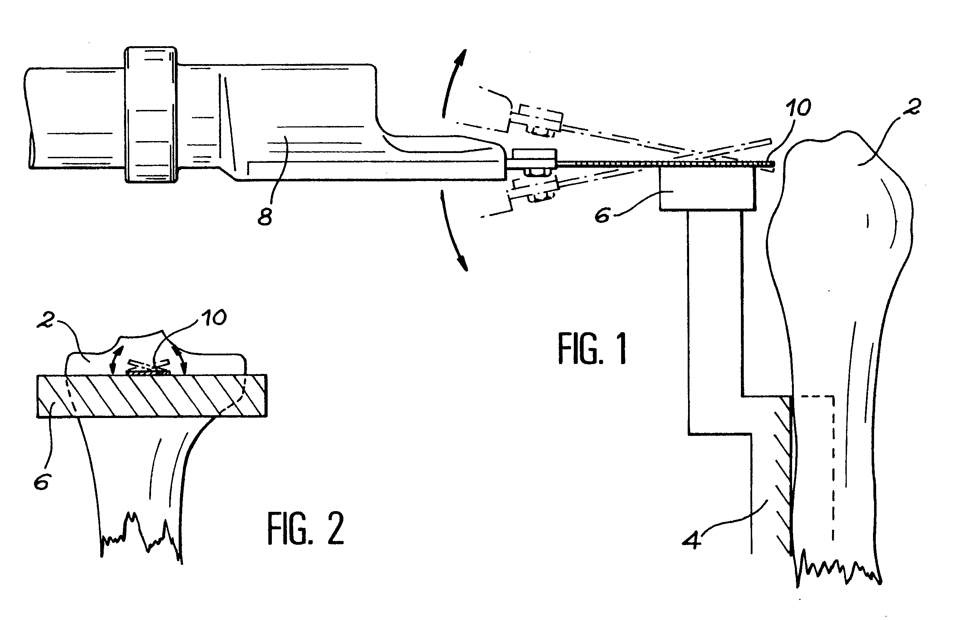

Ce système actuel est décrit en référence aux figures 1 et 2. Il comporte principalement un guide support 6, positionné par rapport à l'os à scier 2, par l'intermédiaire d'un pied de positionnement 4. L'opérateur muni de la lame de scie 10 portée par son bloc-moteur 8, vient appuyer la lame de scie 10 sur le guide support 6. Les quelques positions de la lame de scie 10 représentées en traits mixtes montrent que cette dernière peut bouger de différentes manières, selon plusieurs degrés de liberté. Elle peut être notamment décollée du guide support 6 et inclinée en avant ou en arrière par une rotation du bloc-moteur 8 par rapport au guide support 6.

En référence à la figure 2, on voit que la lame 10 peut également subir un ou deux décollement latéraux par rapport au guide support 6. On constate donc que la coupe ou le sciage de l'os 2 est tributaire de la dextérité de l'opérateur et des différents niveaux de résistance de l'os 2 dans différentes directions.

L'ensemble formé du pied de positionnement 4 et du guide support 6 permet de positionner en hauteur et en inclinaison la lame de scie 10 par rapport à l'os 2, en fonction de l'opération à effectuer. Les valeurs des angles qui définissent l'orientation du plan de coupe ne sont pas nécessairement respectées par le praticien, du fait de la difficulté de maintenir normalement la lame de scie 10 dans ce plan de coupe.

Dans ce cas, le chirurgien tente par une déformation de la lame 10, qui doit nécessairement être flexible, de revenir en appui sur le guide support 6. Ceci entraîne de nombreux inconvénients supplémentaires qui sont les suivants :

- accroissement du frottement de la lame de scie 10 sur le guide support 6, ce qui entraîne une usure rapide de l'un et de l'autre ;
- échauffement de la lame de scie 10, ce qui entraîne une nuisance physiologique ;
- dépôt de corps étrangers, tels que des copeaux métalliques dans les chairs ;
- plan de coupe se déplaçant hors des limites souhaitées.

De plus, l'os présentant des défauts d'homogénéité de densité et de dureté, la lame de scie 10 à tendance à se diriger dans les zones d'effort de coupe minimal, sortant de ce fait du plan de coupe imposé. Enfin, il s'avère très difficile de rescier une tranche de faible épaisseur pour effectuer une éventuelle correction.

Ce dispositif impose donc une certaine flexibilité à la lame de scie pour rattraper les défauts. Pour ne pas augmenter de manière excessive cette flexibilité, la lame ne dépasse pas une certaine longueur. Cette limitation en longueur de la lame limite par conséquent la profondeur de la coupe.

L'invention vise à remédier à tous ces inconvénients.

A cet effet, l'objet de l'invention est un dispositif de positionnement et de guidage de la lame d'une scie chirurgicale, ladite lame étant métallique, allongée, plate, et animée d'un mouvement cyclique dans le plan de la lame par des moyens moteurs placés dans un bloc de la scie, d'où sort la lame de scie.

Le dispositif comprend principalement :
- un pied de positionnement comprenant des moyens de positionnement contre un os à scier, ces moyens de positionnement étant réglables longitudinalement par rapport à l'os ; et
- un guide amovible en rotation par rapport au pied de positionnement, autour d'un axe perpendiculaire au pied de positionnement et destiné à positionner la lame de scie en mouvement par rapport à l'os à scier.

Selon l'invention, le dispositif comprend une coulisse rétractable de positionnement, montée dans le bloc, parallèlement et en dessous de la lame de scie et dont l'extrémité comprend une butée. Conjointement, le guide possède une fente, de largeur supérieure à la largeur de la coulisse et de hauteur légèrement supérieure à l'épaisseur de la coulisse, et dont un bord est destiné à recevoir la butée pour positionner, lorsqu'elle est sortie, la coulisse, et donc la lame de scie à proximité de l'os, et guider la lame de scie pendant son usinage, des moyens élastiques étant prévus pour effectuer le retrait progressif en translation de la coulisse et permettre ainsi à la lame de scie de progresser à travers l'os.

Les qualités de la coupe sont améliorées, le temps de travail diminué, et le mode opératoire facilité. De plus, la lame de scie ne s'échauffe pas, et sa durée de vie est augmentée.

De préférence, la butée est constituée d'une partie triangulaire en saillie dont le sommet vient en buté contre le rebord de la fente.

Une réalisation préférentielle du dispositif selon l'invention prévoit un pied de positionnement avec un corps allongé, les moyens de positionnement du pied étant des vés montés coulissant dans une fente longitudinale placée dans le corps allongé.

Selon un aspect de l'invention, des moyens d'inclinaison du guide sont prévus et sont constitués de premiers trous pratiqués dans le guide et de deuxièmes trous pratiqués dans le corps de façon à ce que chaque premier trou vienne en correspondance avec un deuxième trou pour un angle d'inclinaison déterminé du guide par rapport au pied de positionnement et par conséquent, par rapport à l'os.

Selon un aspect de l'invention, des moyens de réglage de la hauteur de coupe sont prévus et comprennent un palpeur, fixé temporairement sur le guide pour venir en butée sur la surface de la partie de l'os à couper, et possédant des moyens d'affichage du déplacement longitudinal du pied de positionnement par rapport à l'os.

L'invention et toutes ses caractéristiques techniques seront mieux comprises à la lecture de la description qui suit, annexée des figures représentant respectivement :

- figures 1 et 2, déjà décrites, une mise en oeuvre d'un dispositif selon l'art antérieur ;
- figure 3, le dispositif selon l'invention ;
- figure 4, une vue latérale de la lame de scie, de son bloc-moteur et de la coulisse rétractable selon l'invention ;
- figures 5 et 6, le positionnement du guide de la lame de scie ;
- figure 7, les différents mouvements possibles de la lame de scie.

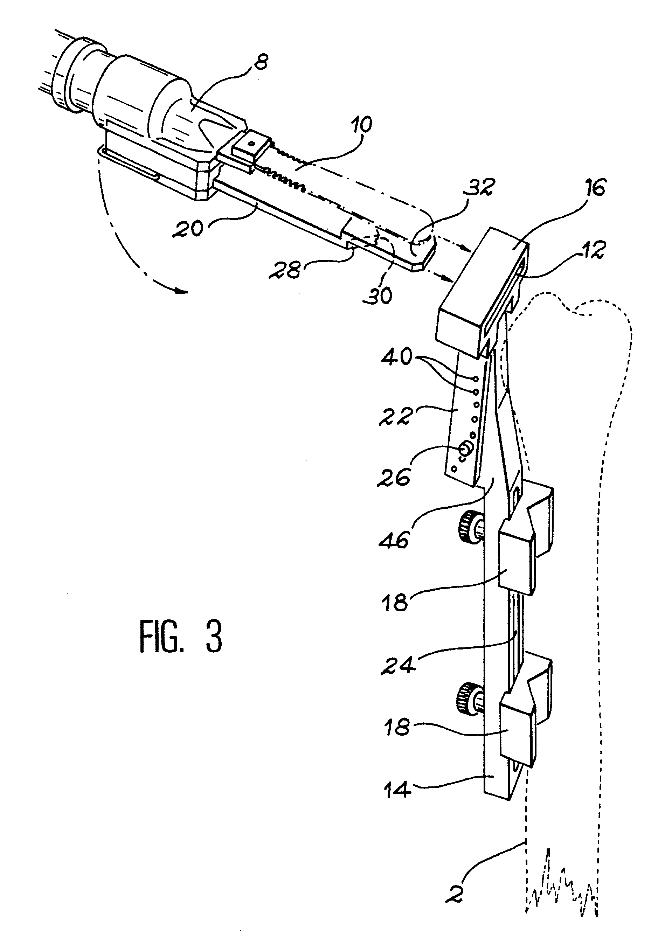

En référence à la figure 3, l'invention est particulièrement bien adaptée à la pose d'une prothèse du genou. La réalisation décrite maintenant est conçue, en particulier, pour scier le bout supérieur du tibia.

Le dispositif selon l'invention reprend des éléments connus de positionnement du guide support de la lame de scie. Plus exactement, on utilise un pied de positionnement 14 placé contre un os 2, en l'occurrence, un tibia. Le positionnement en hauteur se fait à l'aide de moyens de positionnement 18. Ces derniers sont de préférence constitués de deux vés 18, coulissant dans une fente longitudinale 24, pratiquée dans le pied de positionnement 14 qui est un corps allongé.

On utilise également un guide 16 de la lame de scie 10. Celui-ci est positionné en rotation par rapport au pied de positionnement 14 autour d'un axe, non représenté, perpendiculaire au pied de positionnement 14. Le guide 16 est donc réglable en hauteur, par l'intermédiaire des vés de positionnement 18, et est réglable en inclinaison de par son montage tournant autour de l'axe de rotation.

La caractéristique principale du dispositif selon l'invention repose sur l'utilisation d'une fente 12, pratiquée dans le guide de positionnement 16 et de l'utilisation d'une coulisse 20, rétractable dans le bloc 8 porteur de la lame de scie 10. La fente 12 est destinée à recevoir la coulisse rétractable 20, en vue d'assurer le positionnement du bloc 8, et par conséquent le positionnement de la scie 10. En effet, cette coulisse rétractable 20 est montée, parallèlement et en dessous de la lame de scie 10. Ce coulissement longitudinal de la coulisse 20, dans la direction de l'orientation de la lame de scie 10, est le seul degré de liberté de mouvement de cette coulisse rétractable 20 par rapport au bloc moteur 8. Une fois placée dans la fente 12, elle positionne donc le bloc 8 selon cinq degrés de liberté.

Une fois le guide 16 positionné par rapport à l'os 2, la fente 12 débouche contre l'os 2. Comme elle est destinée à recevoir la coulisse rétractable 20, sa largeur est supérieure à la largeur de la coulisse 20. Le positionnement de la lame de scie 10 devant être particulièrement précis en hauteur, la hauteur de la fente 12 est très légèrement supérieure à la hauteur de la coulisse rétractable 20, de manière à ce qu'il ne reste qu'un jeu minimal entre ces deux éléments.

De manière à parfaire ce positionnement de la coulisse 20 dans la fente 12, la coulisse 20 possède, en dessous de son extrémité 32 et légèrement en retrait, une butée 28. Celle-ci a de préférence la forme d'un triangle avec un sommet 30 faisant saillie vers l'extrémité 32 de la lame et destiné à venir en contact avec un rebord de la fente 12. Cette butée 28 assure ainsi, lorsque la coulisse rétractable 20 est sortie, le réglage de la distance de cette coulisse 20 par rapport à l'os à scier 2. Il s'ensuit, un positionnement de la lame de scie 10 par rapport à l'os à scier 2.

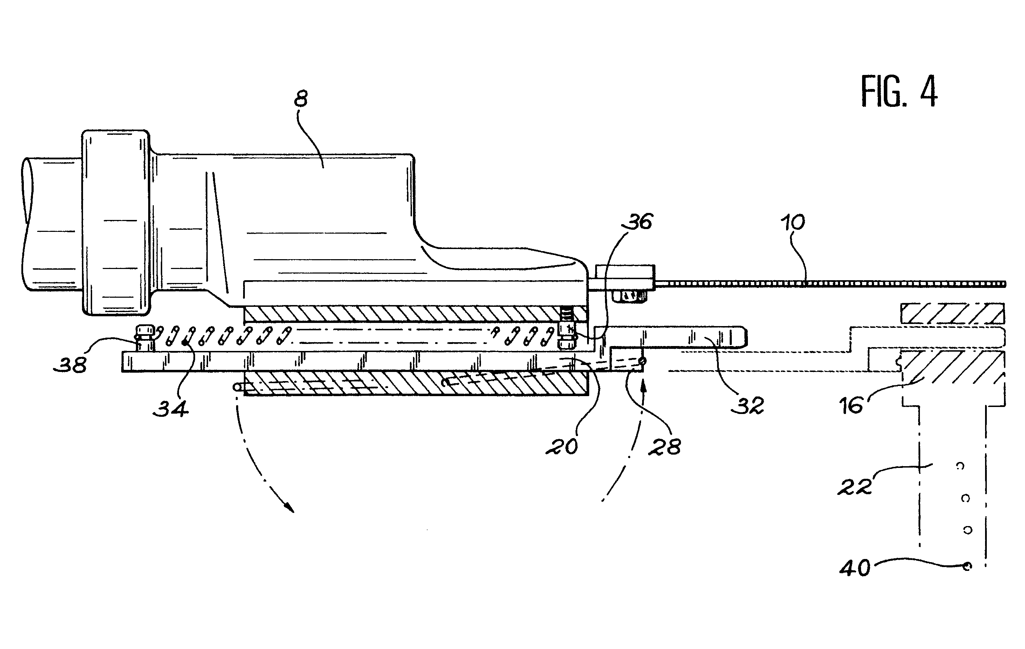

La coulisse rétractable 20 est maintenue sortie, grâce à des moyens élastiques mieux représentés sur la figure 4. La poussée exercée par ces moyens élastiques sur la coulisse rétractable 20 est de faible valeur, de manière à ce que, une fois en butée, la coulisse rétractable 20 puisse rentrer dans le bloc 8 de la scie, lorsque l'opérateur exerce une poussée sur ce bloc 8 en direction de l'os. Ces moyens élastiques peuvent être constitués par exemple d'un ressort 34, fixé d'une part à un premier point de fixation 36 solidaire du bloc 8, et dont l'autre extrémité est solidaire d'un deuxième point de fixation 38, lui-même solidaire de la coulisse rétractable 20.

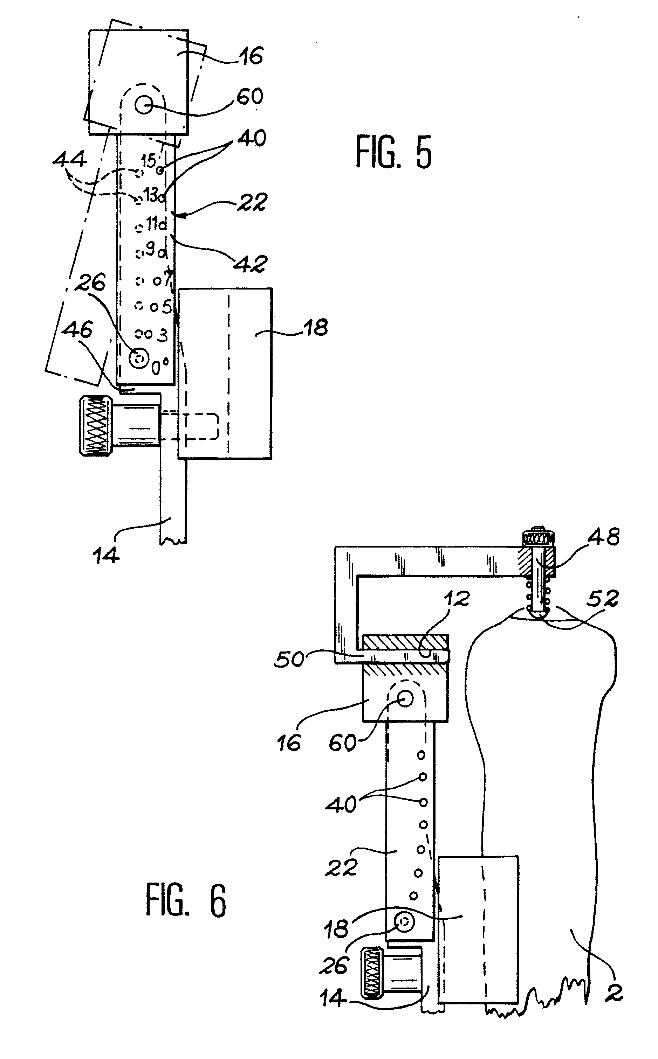

En référence à la figure 5, le guide 16 possède des moyens de sélection de son inclinaison autour de son axe de rotation repéré 60. Ceux-ci sont constitués d'une première série de premiers trous 40, pratiqués dans la partie inférieure 42 du guide 16. Ils sont également constitués d'une deuxième série de deuxième trous 44, pratiqués dans la partie supérieure du pied de positionnement 14. Ces deux séries ont le même nombre de trous, et sont disposées de manière à ce que chaque premier trou 40 de la première série, puisse venir en correspondance avec un deuxième trou 44 de la deuxième série, avec une inclinaison relative déterminée entre le guide 16 et le pied de positionnement 14. On voit en particulier que la première série est constituée de huit trous disposés en arc de cercle dans la partie inférieure 42 du guide 16. La deuxième série comporte également huit trous 44 et est disposée selon une ligne droite. Une telle disposition permet d'obtenir sept angles d'inclinaison différents, en l'occurrence 0, 3, 5, 7, 9, 11, 13, 15°. Une tige 26 introduite dans chacun des deux trous d'un couple, choisi en fonction de l'inclinaison à obtenir, permet de maintenir en correspondance ces deux trous.

L'orientation angulaire du guide 16 est obtenue, comme on l'a vu précédemment, à l'aide des deux séries de premiers trous 40 et de deuxièmes trous 44, la tige 26 venant maintenir un couple de deux trous en correspondance.

En référence à la figure 6, les moyens de positionnement en hauteur du pied de positionnement 14 peuvent être complétés d'un palpeur 48 positionné de manière à venir en butée sur le sommet de l'os à scier 2. Ce palpeur 48 est monté solidaire du guide 16. On utilise de préférence la fente 12 pour y introduire une languette 50 solidaire du palpeur 48, l'ajustement de cette languette 50 dans la fente 12 étant très précis. Ce réglage en hauteur est particulièrement adapté pour effectuer une coupe du sommet du tibia, c'est-à-dire une résection du plateau tibial. Une telle opération peut s'effectuer de la manière suivante.

La distance séparant la coulisse rétractable 20 et la lame de scie 10 étant constante, de l'ordre de 10 mm, et connaissant la position théorique du plan de la coupe à effectuer dans l'os 2, il est facile de déterminer le positionnement longitudinal du pied de positionnement 14 que l'on doit obtenir. Le palpeur à ressort 48 est mis en contact par sa touche 52 avec l'os 2. Quand le ressort du palpeur 48 n'est pas comprimé, le plan de la lame de scie 10 se trouve à la même cote que la touche 52. L'ensemble du pied de positionnement 14 et du palpeur 48 est progressivement descendu, ce qui a pour effet de comprimer le ressort du palpeur 48. Ce dernier étant de préférence équipe de moyen d'affichage d'un déplacement longitudinal, il est donc possible de positionner le futur plan de coupe par rapport au sommet du tibia. Les moyens d'affichage du palpeur 48 sont de préférence des graduations apparaissant sur la tige du palpeur 48. Une fois cet ensemble positionné contre l'os 2, le palpeur 48 et sa languette 50 sont retirés de la fente 12.

On fait remarquer que, dans le cas d'une opération au tibia, le pied de positionnement peut être placé contre le tibia, à même la peau du malade, et étant donné la très faible épaisseur de chair existant entre la surface de la peau et le tibia.

L'ensemble de positionnement étant maintenu fermement contre le tibia, l'opérateur saisit le bloc de scie 8, et introduit la languette rétractable 20 dans la fente 12 du guide 16, jusqu'à ce que la pointe 30 de la butée 28 vienne en contact avec un rebord inférieur de la fente 12. A ce stade, la lame de scie 10 se trouve à quelques millimètres de l'os à scier 2, et est parfaitement positionnée dans l'espace par rapport à celui-ci, en fonction de la coupe à effectuer.

Les moyens moteurs de la lame de scie 10 sont alors mis en route pour obtenir les différents mouvements de celle-ci.

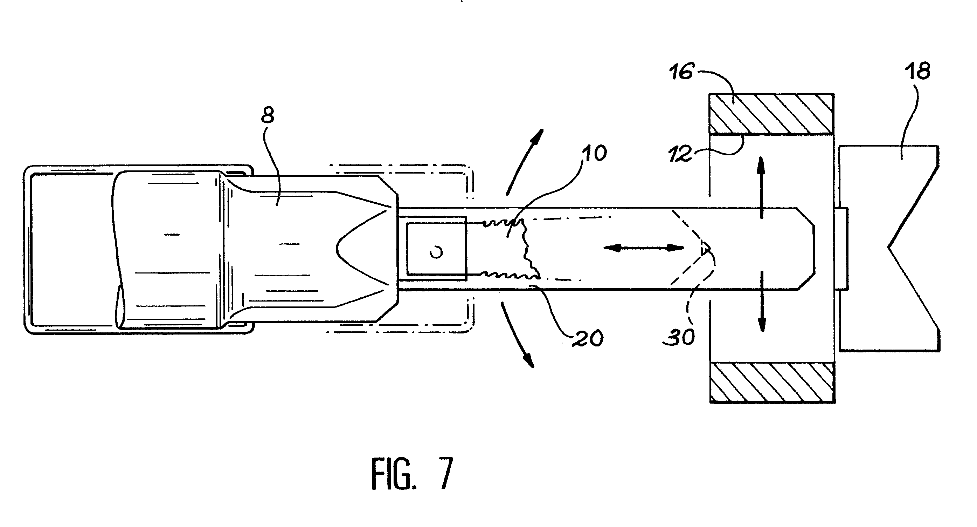

En référence à la figure 7, la lame de scie 10 est actionnée selon plusieurs directions. Elle peut être actionnée longitudinalement, c'est-à-dire parallèlement à son axe, et latéralement. Elle est de préférence actionnée selon un cycle triangulaire qui est une combinaison de deux mouvements alternatifs selon ces deux directions perpendiculaires. On voit que la largeur de la fente 12 permet des mouvements latéraux de la lame de scie 10. La coulisse rétractable 20 étant maintenue en hauteur dans la fente 12, la lame de scie 10 ne peut pas avoir de mouvements verticaux. Tous les mouvements de la lame de scie 10 ont lieu dans le plan de cette lame de scie.

La poussée exercée par l'opérateur a tendance à comprimer les moyens élastiques, en l'occurrence le ressort 34 de la figure 4. Cette poussée de la lame de scie 10, en combinaison avec ses mouvements, la fait pénétrer dans l'os. Le sciage peut ainsi s'effectuer jusqu'à une traversée complète de l'os 2. Si l'opérateur relâche son effort de poussée, le ressort 34 maintient la coulisse rétractable 20 dans sa position en butée dans la fente 12 et garantit la liaison de l'ensemble de la lame de scie 10, du bloc et de l'ensemble de positionnement, dans le cas d'un recul de la lame de scie 10.

Ce dispositif a de nombreux avantages, à commencer par la lame de scie. En effet, la longueur de la lame de scie peut être augmentée. Elle peut en particulier passer de 85 à 100 mm. L'explication vient du fait qu'avec le dispositif de l'art antérieur, l'opérateur éprouve des difficultés à maintenir dans sa position de sciage la lame de scie, car il est obligé de compenser les différents défauts d'homogénéité de la dureté de l'os. La lame de scie doit être d'une flexibilité déterminée.

Au contraire, selon l'invention la lame de scie doit avoir une rigidité maximale pour vaincre les zones de dureté hétérogène dans lesquelles la lame pourrait se déformer. Il faut que dans tous les cas, la laine de scie reste parallèle au plan de coupe.

Un tel dispositif peut effectuer des coupes ayant toutes les qualités requises au niveau de la planéité, concernant l'état de surface et les cotes dimensionnelles.

Avec le dispositif selon l'invention, on évite des dépôts de corps étrangers dans les chairs, et en particulier, de la limaille due au frottement de la lame de scie contre un guide du type de ceux utilisés jusqu'à présent.

Du fait de l'absence de contact de la lame de scie avec un élément de guidage, celle-ci ne s'échauffe pas. Ceci a pour conséquence de ne pas échauffer l'os scié.

La longévité de la lame de scie et des différents ensembles de positionnement de guide est accrue.

L'opération de coupe étant facilitée pour l'opérateur, on obtient un gain de temps de celle-ci.

Le mode de réalisation qui vient d'être décrit s'applique à une résection du plateau tibial.

D'autres moyens de positionnement d'un guide possédant une fente 12 peuvent être conçus pour s'adapter à d'autres os, et en particulier au fémur, de manière à pouvoir effectuer la pose complète d'une prothèse du genou avec un dispositif selon l'invention.

On peut envisager également que la fente ne soit pas parallèle à la lame de scie. Elle peut être aussi perpendiculaire à celle-ci, la coulisse étant alors terminée par une extrémité orientée perpendiculairement au reste de la coulisse. Cette disposition peut aussi bien assurer la position de la lame de scie.

**Revendications**

1. Dispositif de positionnement et de guidage de la lame d'une scie chirurgicale, la lame de scie (10) étant métallique, allongée, plate, animée d'un mouvement cyclique dans le plan de la lame de scie (10) par des moyens moteurs placés dans un bloc (8) de la scie et d'où sort la lame de scie (10), le dispositif comprenant :
- un pied de positionnement (14) comprenant des moyens de positionnement contre un os à scier (2), ces moyens de positionnement étant réglables longitudinalement par rapport à l'os (2) ; et
- un guide (16) amovible en rotation par rapport au pied de positionnement (14) autour d'un axe (26) perpendiculaire au pied de positionnement (14) destiné à positionner la lame de scie (10), en mouvement, par rapport à l'os à scier (2),
le dispositif étant caractérisé en ce qu'il comprend une coulisse rétractable (20) montée dans le bloc (8) parallèlement et en dessous de la lame de scie (10) et dont l'extrémité (32) comprend une butée (28) et en ce que le guide (16) possède une fente (12), de largeur supérieure à la largeur de la coulisse (20) et de hauteur très légèrement supérieure à l'épaisseur de la coulisse (20) et dont un rebord est destiné à recevoir la butée (28) pour positionner, lorsqu'elle est sortie, la coulisse (20) et par conséquent, la lame de scie (10) à proximité de l'os (2) et guider la lame de scie (10) pendant son usinage, des moyens élastiques (34) étant prévus pour effectuer le retrait progressif en translation de la coulisse (20) et permettre à la lame de scie (10) de progresser à travers l'os (2).

2. Dispositif selon la revendication 1, caractérisé en ce que la butée (28) est constituée d'une partie en saillie triangulaire dont le sommet (30) vient en butée contre le rebord de la fente (12).

3. Dispositif selon la revendication 1, caractérisé en ce que le pied de positionnement (14) est un corps allongé, les moyens de positionnement de ce pied (14) étant des vés (18) coulissant dans une fente longitudinale (24) pratiquée dans le pied de positionnement (14).

4. Dispositif selon la revendication 1, caractérisé en ce que des moyens de sélection de l'inclinaison du guide (16) sont prévus et sont constitués d'une série de premiers trous (40) pratiqués dans une partie inférieure (42) du guide (16) et d'une série de deuxièmes trous (44) pratiqués dans le pied de positionnement (14), de façon à ce que chaque premier trou (40) puisse venir en correspondance avec un deuxième trou (44) et solidariser à l'aide d'une clavette (26) pour un angle d'inclinaison déterminé du guide (16) par rapport au pied de positionnement (14).

5. Dispositif selon la revendication 1, caractérisé en ce qu'il comprend des moyens de réglage de la hauteur de coupe, comprenant un palpeur (48) fixé temporairement au guide (16) pour venir en butée sur la surface de la partie d'os à scier (2) et possédant des moyens d'affichage du déplacement longitudinal du pied de positionnement (14) le long de l'os (2).

FIG. 1

FIG. 2

6

FIG. 3

FIG. 4

EP 0 415 837 A2

FIG. 5

FIG. 6

FIG. 7